# EUROPEAN PATENT SPECIFICATION

(11) **EP 2 265 617 B1**
(45) Date of publication and mention of the grant of the patent: **02.07.2014**
(21) Application number: 08806244.3
(22) Date of filing: 12.09.2008
(51) Int. Cl.: C07D 493/18

(54) **SOLVENTS**
LÖSUNGSMITTEL
SOLVANTS

(30) Priority: 12.09.2007 GB 0718269
(43) Date of publication of application: 29.12.2010
(73) Proprietor: Bioniqs Limited, North Yorkshire (GB)
(72) Inventor: WALKER, Adam, Grantham Lincolnshire NG32 3LZ (GB)
(74) Representative: Gilholm, Stephen Philip
(86) International application number: PCT/GB2008/003085
(87) International publication number: WO 2009/034329

(56) References cited:
- WO-A-2007/110637
- GB-A- 2 412 912
- GB-A- 2 430 675
- ANDERSON JARED L ET AL: "Characterizing ionic liquids on the basis of multiple solvation interactions" JOURNAL OF THE AMERICAN CHEMICAL SOCIETY, AMERICAN CHEMICAL SOCIETY, WASHINGTON, DC.; US, US, vol. 124, no. 47, 27 November 2002 (2002-11-27), pages 14247-14254, XP002267362 ISSN: 0002-7863
- POOLE C F ET AL: "ORGANIC SALTS, LIQUID AT ROOM TEMPERATURE, AS MOBILE PHASES IN LIQUID CHROMATOGRAPHY" JOURNAL OF CHROMATOGRAPHY, ELSEVIER SCIENCE PUBLISHERS B.V. AMSTERDAM, NL, vol. 352, 1 January 1986 (1986-01-01), pages 407-425, XP002346176 ISSN: 0021-9673
- ARMSTRONG D W ET AL: "IONIC LIQUIDS AS MATRIXES FOR MATRIX-ASSISTED LASER DESORPTION/IONIZATION MASS SPECTROMETRY" ANALYTICAL CHEMISTRY, AMERICAN CHEMICAL SOCIETY. COLUMBUS, US, vol. 73, no. 15, 1 August 2001 (2001-08-01), pages 3679-3686, XP001156233 ISSN: 0003-2700

## Description

### FIELD OF THE INVENTION

The present invention relates to the use of certain organic compounds, as solvents. In particular, the invention relates to the use certain organic liquids as solvents.

### BACKGROUND

Ionic liquids are compounds which are composed predominantly of ions but are in liquid form, generally having a melting point below ambient temperature. They arise from combinations of suitable ions in which lattice energy and melting point are abnormally low.

Ionic liquids can possess a number of unusual properties, including negligible vapour pressure, high solubilising power and a broad liquid temperature range which render them interesting alternatives to conventional liquids, such as conventional organic solvents hereinbefore described.

Tamar, et al in "Formation of Amphiphile Self Assembly Phases in Protic Ionic Liquids" J. Phys. Chem. B 2007, 111, 4082-4088; describes a range of protic ionic liquids comprising primary and secondary alkyl ammonium salts, e.g. methyl ammonium formate, and the like, which exhibit self-assembly liquid crystal behaviour.

Torriero, *et al* in a poster entitled "Electrochemistry in Protic Ionic Liquids" at the COIL2 conference in Yokohama in August 2007 have described the tertiary ammonium salt, triethylammonium acetate, as being interesting as an anhydrous proton conductor in hydrogen-oxygen fuel cells.

UK Patent application No. 2412912 describes an ionic liquid comprising an anion and a cation wherein the cation is a nitrogen containing cation of the formula (I) N+HRR'R" (I) in which R may be a hydrocarbyl group and R' and R" each represent H or R. However, the specific ionic liquids described therein are relatively viscous and polar.

We have now surprisingly found a group of protic ionic liquids that are advantageous as solvents, for example, in the processing of organic compounds.

### SUMMARY OF INVENTION

Therefore, according to a first aspect of the invention we provide the use of a trialkylammonium salt as a protic ionic liquid wherein the trialkylammonium moiety is selected from the group consisting of N,N-dimethylethylammonium acetate; N,N-dimethylethylammonium propionate; N,N-dimethylethylammonium butyrate; N,N-dimethylethylammonium hexanoate; N,N-dimethylethylammonium heptanoate; N,N-dimethylethylammonium octanoate; N,N-dimethylethylammonium crotonate; N,N-dimethylethylammonium phenylacetate; N,N-dimethylethylammonium 2,2-dimethylbutyrate; N,N-dimethylbutylammonium acetate; N,N-dimethylbutylammonium propionate; N,N-dimethylbutylammonium butyrate; N,N-dimethylbutylammonium hexanoate; N,N-dimethylbutylammonium heptanoate; N,N-dimethylbutylammonium octanoate; N,N-dimethylbutylammonium benzoate; N,N-dimethylbutylammonium phenylacetate; N,N-dimethylbutylammonium 2,2-dimethylbutyrate; N,N-dimethylbutylammonium 2-hexyldecanoate; N,N-dimethylbutylammonium 2-methylvalerate; N,N-dimethylbutylammonium decanoate; N,N-dimethylhexylammonium acetate; N5N-dimethyldodecylammonium propionate; N,N-dimethyldodecylammonium butyrate; N,N-dimethyldodecylammonium hexanoate; N,N-dimethyldodecylammonium heptanoate; N,N-dimethyldodecylammonium octanoate; N-methylpiperidinium acetate; N-methylpiperidinium propionate; N-methylpiperidinium butyrate; N-methylpiperidinium hexanoate; N-methylpiperidinium heptanoate; N,N,N-triethylammonium propionate; N,N,N-triethylammonium butyrate; N,N,N-triethylammonium hexanoate; N,N,N-triethylammonium crotonate; N,N-diisopropyl-N-ethylammonium butyrate; N,N-diisopropyl-N-ethylammonium octanoate; N,N,N-trioctylammonium propionate; N,N,N-trioctylammonium 2-oxobutyrate; N,N,N-trioctylammonium 3-toluate; N,N,N-triisobutylammonium butyrate; trimethylammonium formate; trimethylammonium acetate; trimethylammonium propionate; trimethylammonium butyrate; trimethylammonium valerate; trimethylammonium hexanoate; trimethylammonium heptanoate; trimethylammonium octanoate; trimethylammonium glycolate; N,N-dimethylethylammonium formate; N,N-dimethylethylammonium valerate; N,N-dimethylethylammonium trans-2-hexenoate; N,N-dimethylpropylammonium formate; N,N-dimethylpropylammonium acetate; N,N-dimethylpropylammonium propionate; N,N-dimethylpropylammonium butyrate; N,N-dimethylpropylammonium valerate; N,N-dimethylpropylammonium hexanoate; N,N-dimethylpropylammonium heptanoate; N,N-dimethylpropylammonium octanoate; N,N-dimethylbutylammonium formate; N,N-dimethylhexylammonium valerate; N,N-dimethyldecylammonium butyrate; N,N-dimethyldecylammonium valerate; N,N-dimethyldecylammonium hexanoate; N,N-dimethyldecylammonium heptanoate; N,N-dimethyldecylammonium octanoate; N,N-dimethyldecylammonium pivalate; N,N-dimethyldodecylammonium valerate; N-methyldiethylammonium acetate; N-methyldiethylammonium propionate; N-methyldiethylammonium butyrate; N-methyldiethylammonium valerate; N-methyldiethylammonium hexanoate; N-methyldiethylammonium heptanoate; N-methyldiethylammonium octanoate; N-methyldipropylammonium acetate; N-methyldipropylammonium propionate; N-methyldipropylammonium butyrate; N-methyldipropylammonium valerate; N-methyldipropylammonium hexanoate; N-methyldipropylammonium heptanoate; N-methyldipropylammonium octanoate; N-methyldibutylammonium acetate; N-methyldibutylammonium propionate; N-methyldibutylammonium butyrate; N-methyldibutylammonium valerate; N-methyldibutylammonium hexanoate; N-methyldibutylammonium heptanoate; N-methyldibutylammonium octanoate; N-methyldicyclohexylammonium propionate; N-methyldicyclohexylammonium butyrate; N-methyldicyclohexylammonium valerate; N-methyldicyclohexylammonium hexanoate; N-methyldicyclohexylammonium heptanoate; N-methyldicyclohexylammonium octanoate; N-ethyldiisopropylammonium formate; N-ethyldiisopropylammonium valerate; N-ethyldiisopropylammonium hexanoate; N-ethyldiisopropylammonium heptanoate; N-ethyldiisopropylammonium 2-methylvalerate; N-ethyldiisopropylammonium 2-hexyldecanoate; triethylammonium valerate; triethylammonium heptanoate; triethylammonium octanoate; triethylammonium methanesulfonate; tripropylammonium propionate; tripropylammonium butyrate; tripropylammonium valerate; tripropylammonium hexanoate; tripropylammonium heptanoate; tripropylammonium octanoate; tributylammonium propionate; tributylammonium butyrate; tributylammonium valerate; tributylammonium hexanoate; tributylammonium heptanoate; tributylammonium octanoate; triisobutylammonium valerate; triisobutylammonium hexanoate; triisobutylammonium heptanoate; triisobutylammonium octanoate; triisobutylammonium crotonate; triisobutylammonium 2,2-dimethylbutyrate; triisobutylammonium 2-methylvalerate; triisobutylammonium decanoate; trihexylammonium butyrate; trihexylammonium valerate; trihexylammonium hexanoate; trihexylammonium heptanoate; trihexylammonium octanoate; trioctylammonium acetate; trioctylammonium butyrate; trioctylammonium valerate; trioctylammonium hexanoate; trioctylammonium heptanoate; trioctylammonium octanoate; trioctylammonium nonanoate; trioctylammonium decanoate; trioctylammonium crotonate; trioctylammonium trans-2-hexenoate; trioctylammonium 2-butyloctanoate; trioctylammonium benzoate; trioctylammonium phenylacetate; triisooctylammonium propionate; triisooctylammonium butyrate; triisooctylammonium valerate; triisooctylammonium hexanoate; triisooctylammonium heptanoate; triisooctylammonium octanoate; N-methylpiperidinium formate; N-methylpiperidinium valerate; N-methylpyrrolidinium formate; N-methylpyrrolidinium acetate; N-methylpyrrolidinium propionate; N-methylpyrrolidinium butyrate; N-methylpyrrolidinium valerate; N-methylpyrrolidinium hexanoate; N-methylpyrrolidinium heptanoate; and N-methylpyrrolidinium octanoate. It is within the scope of the present invention to provide any one of the trialkylammonium moieties listed above.

In particular we provide the use of a protic ionic liquid comprising a trialkylammonium salt as a solvent in the manufacture of an organic compound.

In a further aspect of the invention the salts comprise an trialkylammonium moiety selected form the group consisting of are trimethylammonium, triethylammonium, tripropylammonium, tributylammonium, triisobutylammonium, triamylanimonium, trihexylammonium, triheptylammonium, trioctylammonium, dimethylethylammonium, dimethylpropylammonium, dimethylbutylammonium, dimethylamylammonium, dimethylhexylammonium, dimethylheptylammonium, dimethyloctylammonium, dimethylnonylammonium, dimethyldecylammonium, dimethylundecylammonium, dimethyldodecylammonium, dimethyltridecyl ammonium, dimethyltetradecylammonium, dimethylpentadecylammonium, dimethylhexadecylammonium, dimethylisopropylammonium, dimethylisobutyl ammonium, dimethylisoamylammonium, dimethylisohexylammonium, dimethylisoheptylammonium, dimethylisooctylammonium, dimethyl tert-butylammonium, dimethyl tert-amylammonium, dimethylcyclopentylammonium, dimethylcyclohexylammonium, dimethylcycloheptylammonium, dimethylcyclooctyl ammonium, methyldiethylammonium, methyldipropylammonium, methyldibutylammonium, methyldiamylammonium, methyldihexylammonium, methyldiheptylammonium, methyldioctylammonium, methyldiisopropylammonium, methyldiisobutylammonium, methyldiisoamylammonium, methyldi-tert-butylammonium, methyldi-tert-amyl-ammonium, methylpyrrolidinium, methylpiperidinium, methyldicyclohexylammonium, diethylpropylammonium, diethylbutylammonium, diethylamylammonium, diethylhexylammonium, diethylheptylammonium, diethyloctylammonium, diethylnonylammonium, diethyldecylammonium, diethylundecylammonium, diethyldodecylammonium, diethyltridecylammonium, diethyltetradecylammonium, diethylpentadecylammonium, diethylhexadecylammonium, diethylisopropylammonium, diethylisobutylammonium, diethylisoamylammonium, diethylisohexylammonium, diethylisoheptylammonium, diethylisooctylammonium, diethyl tert-butylammonium, diethyl tert-amylammonium, diethylcyclopentylammonium, diethylcyclohexylammonium, diethylcycloheptyl ammonium, diethylcyclooctylammonium, ethyldipropylammonium, ethyldibutylammonium, ethyldiamylammonium, ethyldihexylammonium, ethyldiheptylammonium, ethyldioctylammonium, ethyldiisopropylammonium, ethyldiisobutylammonium, ethyldiisoamylammonium, ethyldi-tert-butylammonium, ethyldi-tert-amyl-ammonium, ethylpyrrolidinium, ethylpiperidinium and ethyldicyclohexylammonium.

An especially preferred group o slats are those in which the trialkylammonium moiety is selected from the group consisting of N,N-dimethylethylammonium acetate; N,N-dimethylethylammonium propionate; N,N-dimethylethylammonium butyrate; N,N-dimethylethylammonium hexanoate; N,N-dimethylethylammonium heptanoate; N,N-dimethylethylammonium octanoate; N,N-dimethylethylammonium crotonate; N,N-dimethylethylammonium phenylacetate; N,N-dimethylethylammonium 2,2-dimethylbutyrate; N,N-dimethylbutylammonium acetate; N,N-dimethylbutylammonium propionate; N,N-dimethylbutylammonium butyrate; N,N-dimethylbutylammonium hexanoate; N,N-dimethylbutylammonium heptanoate; N,N-dimethylbutylammonium octanoate; N,N-dimethylbutylammonium benzoate; N,N-dimethylbutylammonium phenylacetate; N,N-dimethylbutylammonium 2,2-dimethylbutyrate; N,N-dimethylbutylammonium 2-hexyldecanoate; N,N-dimethylbutylammonium 2-methylvalerate; N,N-dimethylbutylammonium decanoate; N,N-dimethylhexylammonium acetate; N,N-dimethyldodecylammonium propionate; N,N-dimethyldodecylammonium butyrate; N,N-dimethyldodecylammonium hexanoate; N,N-dimethyldodecylammonium heptanoate; N,N-dimethyldodecylammonium octanoate; N-methylpiperidinium acetate; N-methylpiperidinium propionate; N-methylpiperidinium butyrate; N-methylpiperidinium hexanoate; N-methylpiperidinium heptanoate; N,N,N-triethylammonium propionate; N,N,N-triethylammonium butyrate; N,N,N-triethylammonium hexanoate; N,N,N-triethylammonium crotonate; N,N-diisopropyl-N-ethylammonium butyrate; N,N-diisopropyl-N-ethylammonium octanoate; N,N,N-trioctylammonium propionate; N,N,N-trioctylammonium 2-oxobutyrate; N,N,N-trioctylammonium 3-toluate; and N,N,N-triisobutylammonium butyrate.

The salt may comprise a conventionally known acid addition salt, thus the alkyl ammonium moiety may be in association with any anion or combination of anions (with non-halogenated organic carboxylates and sulfonates being especially preferred), wherein the resulting salt has a melting point below 100°C (most preferably with a melting point below 25°C and a viscosity at 25°C of less than 250cP). Preferred salts which may be mentioned are the salts of an organic acid, e.g. a C1 to 10 carboxylic acid, a substituted C1 to 10 carboxylic acid, or the salt of an inorganic acid, e.g. nitric acid or sulphuric acid. The salts of substituted C1 to 10 carboxylic acids which may be mentioned are, for example, glycolate and lactate. However, the salts of a C1 to 10 (unsubstituted) carboxylic acid are preferred, more preferably salts of a C1 to 6 carboxylic acid and especially salts of a C1 to 4 carboxylic acid. Salts which may be mentioned include formate, acetate, propionate and butyrate. Especially preferred is the propionate salt.

The ionic liquids of the invention are advantageous in that, *inter alia,* they are miscible with a variety of other solvents in varying proportions, such as, water, dimethylsulphoxide, acetonitrile, ethyl acetate, tetrahydrofuran, chloroform, hexane and toluene.

It will be appreciated by one skilled in the art that the manufacture of organic compounds as hereinbefore described may comprise one or more of the purification, recovery, dissolution, extraction, separation, reaction, modification, preparation, processing and/or recycling of organic compounds. In a preferred aspect of the invention the manufacture comprises the extraction of an organic compound, e.g. the extraction of an organic compound from a biomass. The use of the solvent as hereinbefore described may comprise the manufacture of a wide variety of organic compounds, more specifically the use of the invention comprises the manufacture of a terpene as hereinbefore described. For the avoidance of doubt, by the term terpene we include, *inter alia,* carotenes, carotenoids, isoprenoids, prenols, retinoids, steroids, terpenoids. The use of the invention is especially useful in the extraction of naturally occurring compounds, such as the terpenes, including terpenoids.

According to a further aspect of the invention we provide a method of manufacturing an organic compound which comprises the use of a trialkylammonium salt as a solvent.

The method of the invention is advantageous in that, inter alia, protic ionic liquid solvent may be regenerated once used.

We further provide an organic compound manufactured by the method of the invention as hereinbefore described.

The trialkylammonium salts of general formula I;

R¹R²R³NH⁺ X⁻ I

wherein R¹, R² and R³, which may be the same or different, are each alkyl C1 to 10; and
X is a C1 to 10 carboxylate, a substituted C1 to 10 carboxylate or an inorganic acid anion;
are either known, may be prepared by methods known *per se* or may be made by reacting a compound of formula II;

R¹R²R³N II

in which R¹, R² and R³, which may be the same or different, are as hereinbefore defined;
with an acid of formula III;

H X III

in which X is as hereinbefore defined.

Compounds of formulae II and III themselves are either known *per se* or may be made by using conventional methods known in the art.

The invention will now be described by way of example only.

In the examples below, the values π*, α, β and E_{N}^{T} are the Kamlet-Taft and Reichardt solvatochromic polarity values and indicate the specific solvent properties of the individual compounds analysed.

### Examples

### 1. N,N-Dimethylethylammonium acetate

N,N-Dimethylethylamine (1.0mol, 73.14g) was placed in an argon-flushed round-bottomed flask equipped with a magnetic stirrer and cooled to 0°C in an ice bath. Acetic acid (1.00 equiv., 1.0mol, 60.06g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 2.7cP.
Density @ 25°C: 0.90 g/cm³
Refractive Index @ 25°C: 1.412
π*: 0.74
β: 0.94

### 2. N,N-Dimethylethylammonium propionate

N,N-Dimethylethylamine (1.0mol, 73.14g) was placed in an argon-flushed round-bottomed flask equipped with a magnetic stirrer and cooled to 0°C in an ice bath. Propionic acid (1.00 equiv., 1.0mol, 74.08g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 2.2cP
Density @ 25°C: 0.86 g/cm³
Refractive Index @ 25°C: 1.414
π*: 0.69
β: 0.94

### 3. N,N-Dimethylethylammonium butyrate

N,N-Dimethylethylamine (1.0mol, 73.14g) was placed in an argon-flushed round-bottomed flask equipped with a magnetic stirrer and cooled to 0°C in an ice bath. Butyric acid (1.00 equiv., 1.0mol, 88.11g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 2.6cP
Density @ 25°C: 0.86 g/cm³
Refractive Index @ 25°C: 1.411
π*: 0.67
β: 0.95

### 4. N,N-Dimethylethylammonium hexanoate

N,N-Dimethylethylamine (1.0mol, 73.14g) was placed in an argon-flushed round-bottomed flask equipped with a magnetic stirrer and cooled to 0°C in an ice bath. Hexanoic acid (1.00 equiv., 1.0mol, 116.16g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 3.2cP
Density @ 25°C: 0.86 g/cm³
Refractive Index @ 25°C: 1.419
π*: 0.65

### 5. N,N-Dimethylethylammonium heptanoate

N,N-Dimethylethylamine (1.0mol, 73.14g) was placed in an argon-flushed round-bottomed flask equipped with a magnetic stirrer and cooled to 0°C in an ice bath. Heptanoic acid (1.00 equiv., 1.0mol, 130.18g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 3.7cP
Density @ 25°C: 0.85 g/cm³
Refractive Index @ 25°C: 1.421
π*: 0.62

### 6. N,N-Dimethylethylammonium octanoate

N,N-Dimethylethylamine (1.0mol, 73.14g) was placed in an argon-flushed round-bottomed flask equipped with a magnetic stirrer and cooled to 0°C in an ice bath. Octanoic acid (1.00 equiv., 1.0mol, 144.21 g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 4.5cP
Density @ 25°C: 0.85 g/cm³
Refractive Index @ 25°C: 1.426
π*: 0.60

### 7. N,N-Dimethylethylammoniom crotonate

N,N-Dimethylethylamine (1.0mol, 73.14g) was placed in an argon-flushed round-bottomed flask equipped with a magnetic stirrer and cooled to 0°C in an ice bath. Crotonic acid (1.00 equiv., 1.0mol, 86.09g) was added portionwise with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 4.7cP
Density @ 25°C: 0.90 g/cm³
Refractive Index @ 25°C: 1.438
π*: 0.76
β: 0.92

### 8. N,N-Dimethylethylammonium phenylacetate

N,N-Dimethylethylamine (1.0mol, 73.14g) was placed in an argon-flushed round-bottomed flask equipped with a magnetic stirrer and cooled to 0°C in an ice bath. Phenylacetic acid (1.00 equiv., 1.0mol, 136.15g) was added portionwise with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 17.7cP
Density @ 25°C: 1.00 g/cm³
Refractive Index @ 25°C: 1.499
π*: 0.85
β: 0.95

### 9. N,N-Dimethylethylammonium 2,2-dimethylbutyrate

N,N-Dimethylethylamine (1.0mol, 73.14g) was placed in an argon-flushed round-bottomed flask equipped with a magnetic stirrer and cooled to 0°C in an ice bath. 2,2-Dimethylbutyric acid (1.00 equiv., 1.0mol, 116.16g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 3.0cP
Density @ 25°C: 0.85 g/cm³
Refractive Index @ 25°C: 1.414
π*: 0.59
β: 0.86

### 10. N,N-Dimethylbutylammonium acetate

N,N-Dimethylbutylamine (1.0mol, 101.19g) was placed in an argon-flushed round-bottomed flask equipped with a magnetic stirrer and cooled to 0°C in an ice bath. Acetic acid (1.00 equiv., 1.0mol, 60.06g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 4.2cP
Density @ 25°C: 0.86 g/cm³
Refractive Index @ 25°C: 1.414
π*: 0.73
α: 1.29
β: 0.94
E_{N}^{T}: 0.88

### 11. N,N-Dimethylbutylammonium propionate

N,N-Dimethylbutylamine (1.0mol, 101.19g) was placed in an argon-flushed round-bottomed flask equipped with a magnetic stirrer and cooled to 0°C in an ice bath. Propionic acid (1.00 equiv., 1.0mol, 74.08g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 3.7cP
Density @ 25°C: 0.85 g/cm³
Refractive Index @ 25°C: 1.416
π*: 0.66
α: 1.42
β: 0.95
E_{N}^{T}: 0.92

### 12. N,N-Dimethylbutylammonium butyrate

N,N-Dimethylbutylamine (1.0mol, 101.19g) was placed in an argon-flushed round-bottomed flask equipped with a magnetic stirrer and cooled to 0°C in an ice bath. Butyric acid (1.00 equiv., 1.0mol, 88.11g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 4.2 cP
Density @ 25°C: 0.84 g/cm³
Refractive Index @ 25°C: 1.417
π*: 0.64
α: 1.45
β: 0.90
E_{N}^{T}: 0.92

### 13. N,N-Dimethylbutylammonium hexanoate

N,N-Dimethylbutylamine (1.0mol, 101.19g) was placed in an argon-flushed round-bottomed flask equipped with a magnetic stirrer and cooled to 0°C in an ice bath. Hexanoic acid (1.00 equiv., 1.0mol, 116.16g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 5.0cP
Density @ 25°C: 0.84 g/cm³
Refractive Index @ 25°C: 1.423
π*: 0.62
α: 1.50
β: 0.94
E_{N}^{T}: 0.94

### 14. N,N-Dimethylbutylammonium heptanoate

N,N-Dimethylbutylamine (1.0mol, 101.19g) was placed in an argon-flushed round-bottomed flask equipped with a magnetic stirrer and cooled to 0°C in an ice bath. Heptanoic acid (1.00 equiv., 1.0mol, 130.18g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 5.7cP
Density @ 25°C: 0.84 g/cm³
Refractive Index @ 25°C: 1.424
π*: 0.61
α: 1.49
β: 0.83
E_{N}^{T}: 0.93

### 15. N,N-Dimethylbutylammonium octanoate

N,N-Dimethylbutylamine (1.0mol, 101.19g) was placed in an argon-flushed round-bottomed flask equipped with a magnetic stirrer and cooled to 0°C in an ice bath. Octanoic acid (1.00 equiv., 1.0mol, 144.21g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 6.6 cP
Density @ 25°C: 0.83 g/cm³
Refractive Index @ 25°C: 1.426
π*: 0.63
α: 1.49
β: 0.94
E_{N}^{T}: 0.94

### 16. N,N-Dimethylbutylammonium benzoate

N,N-Dimethylbutylamine (1.0mol, 101.19g) was placed in an argon-flushed round-bottomed flask equipped with a magnetic stirrer and cooled to 0°C in an ice bath. Benzoic acid (1.00 equiv., 1.0mol, 122.12g) was added portionwise with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 31.0cP
Density @ 25°C: 0.97 g/cm³
Refractive Index @ 25°C: 1.498
π*: 0.83
α: 1.18
β: 0.90
E_{N}^{T}: 0.86

### 17. N,N-Dimethylbutylammonium phenylacetate

N,N-Dimethylbutylamine (1.0mol, 101.19g) was placed in an argon-flushed round-bottomed flask equipped with a magnetic stirrer and cooled to 0°C in an ice bath. Phenylacetic acid (1.00 equiv., 1.0mol, 136.15g) was added portionwise with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 15.0cP
Density @ 25°C: 0.96 g/cm³
Refractive Index @ 25°C: 1.488
π*: 0.78
α: 1.20
β: 0.97
E_{N}^{T}: 0.85

### 18. N,N-Dimethylbutylammonium 2,2-dimethylbutyrate

N,N-Dimethylbutylamine (1.0mol, 101.19g) was placed in an argon-flushed round-bottomed flask equipped with a magnetic stirrer and cooled to 0°C in an ice bath. 2,2-Dimethylbutyric acid (1.00 equiv., 1.0mol, 116.16g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 5.2cP
Density @ 25°C: 0.84 g/cm³
Refractive Index @ 25°C: 1.420
π*: 0.60
α: 1.60
β: 0.87
E_{N}^{T}: 0.98

### 19. N,N-Dimethylbutylammonium 2-hexyldecanoate

N,N-Dimethylbutylamine (1.0mol, 101.19g) was placed in an argon-flushed round-bottomed flask equipped with a magnetic stirrer and cooled to 0°C in an ice bath. 2-Hexyldecanoic acid (1.00 equiv., 1.0mol, 256.42g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 16.0 cP
Density @ 25°C: 0.84 g/cm³
Refractive Index @ 25°C: 1.439
π*: 0.50
α: 1.61
β: 0.83
E_{N}^{T}: 0.95

### 20. N,N-Dimethylbutylammonium 2-methylvalerate

N,N-Dimethylbutylamine (1.0mol, 101.19g) was placed in an argon-flushed round-bottomed flask equipped with a magnetic stirrer and cooled to 0°C in an ice bath. 2-Methylvaleric acid (1.00 equiv., 1.0mol, 116.16g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 4.5 cP
Density @ 25°C: 0.84 g/cm³
Refractive Index @ 25°C: 1.420
π*: 0.61
α: 1.53
β: 0.97
E_{N}^{T}: 0.95

### 21. N,N-Dimethylbutylammonium decanoate

N,N-Dimethylbutylamine (1.0mol, 101.19g) was placed in an argon-flushed round-bottomed flask equipped with a magnetic stirrer and cooled to 0°C in an ice bath. Decanoic acid (1.00 equiv., 1.0mol, 172.26g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 8.2cP
Density @ 25°C: 0.85 g/cm³
Refractive Index @ 25°C: 1.432
π*: 0.59
α: 1.54
β: 1.10
E_{N}^{T}: 0.95

### 22. N,N-Dimethylhexylammonium acetate

N,N-Dimethylhexylamine (1.0mol, 129.24g) was placed in an argon-flushed round-bottomed flask equipped with a magnetic stirrer and cooled to 0°C in an ice bath. Acetic acid (1.00 equiv., 1.0mol, 60.06g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 4.2cP
Density @ 25°C: 0.85 g/cm³
Refractive Index @ 25°C: 1.419
π*: 0.65
β: 0.96

### 23. N,N-Dimethyldodecylammonium propionate

N,N-Dimethyldodecylamine (1.0mol, 213.40g) was placed in an argon-flushed round-bottomed flask equipped with a magnetic stirrer and cooled to 0°C in an ice bath. Propionic acid (1.00 equiv., 1.0mol, 74.08g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 9.1cP
Density @ 25°C: 0.85 g/cm³
Refractive Index @ 25°C: 1.434
π*: 0.57
α: 1.45
β: 0.83
E_{N}^{T}: 0.90

### 24. N,N-Dimethyldodecylammonium butyrate

N,N-Dimethyldodecylamine (1.0mol, 213.40g) was placed in an argon-flushed round-bottomed flask equipped with a magnetic stirrer and cooled to 0°C in an ice bath. Butyric acid (1.00 equiv., 1.0mol, 88.11g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 9.7cP
Density @ 25°C: 0.84 g/cm³
Refractive Index @ 25°C: 1.435
π*: 0.56
α: 1.44
β: 0.80
E_{N}^{T}: 0.89

### 25. N,N-Dimethyldodecylammonium hexanoate

N,N-Dimethyldodecylamine (1.0mol, 213.40g) was placed in an argon-flushed round-bottomed flask equipped with a magnetic stirrer and cooled to 0°C in an ice bath. Hexanoic acid (1.00 equiv., 1.0mol, 116.16g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 9.6cP
Density @ 25°C: 0.84 g/cm³
Refractive Index @ 25°C: 1.438
π*: 0.53
α: 1.46
β: 0.87
E_{N}^{T}: 0.89

### 26. N,N-Dimethyldodecylammonium heptanoate

N,N-Dimethyldodecylamine (1.0mol, 213.40g) was placed in an argon-flushed round-bottomed flask equipped with a magnetic stirrer and cooled to 0°C in an ice bath. Heptanoic acid (1.00 equiv., 1.0mol, 130.18g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 10.8cP
Density @ 25°C: 0.84 g/cm³
Refractive Index @ 25°C: 1.439
π*: 0.53
α: 1.46
β: 0.85
E_{N}^{T}: 0.89

### 27. N,N-Dimethyldodecylammonium octanoate

N,N-Dimethyldodecylamine (1.0mol, 213.40g) was placed in an argon-flushed round-bottomed flask equipped with a magnetic stirrer and cooled to 0°C in an ice bath. Octanoic acid (1.00 equiv., 1.0mol, 144.21g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 11.9cP
Density @ 25°C: 0.84 g/cm³
Refractive Index @ 25°C: 1.440
π*: 0.53
α: 1.43
β: 0.83
E_{N}^{T}: 0.88

### 28. N-Methylpiperidinium acetate

N-Methylpiperidine (1.0mol, 99.17g) was placed in an argon-flushed round-bottomed flask equipped with a magnetic stirrer and cooled to 0°C in an ice bath. Acetic acid (1.00 equiv., 1.0mol, 60.06g) was run in with stirring at such a rate as to prevent the temperature of the reaction form exceeding 40°C. After the addition of the last of the acid, the reaction was stirred on ice for 25 minutes. The resultant product was a pale yellow liquid with the following properties:
Viscosity @ 25°C: 5.5cP
Density @ 25°C: 0.94 g/cm³
Refractive Index @ 25°C: 1.440
π*: 0.69

### 29. N-Methylpiperidinium propionate

N-Methylpiperidine (1.0mol, 99.17g) was placed in an argon-flushed round-bottomed flask equipped with a magnetic stirrer and cooled to 0°C in an ice bath. Propionic acid (1.00 equiv., 1.0mol, 74.08g) was run in with stirring at such a rate as to prevent the temperature of the reaction form exceeding 40°C. After the addition of the last of the acid, the reaction was stirred on ice for 25 minutes. The resultant product was a pale yellow liquid with the following properties:
Viscosity @ 25°C: 5.2cP
Density @ 25°C: 0.93 g/cm³
Refractive Index @ 25°C: 1.440
π*: 0.69

### 30. N-Methylpiperidinium butyrate

N-Methylpiperidine (1.0mol, 99.17g) was placed in an argon-flushed round-bottomed flask equipped with a magnetic stirrer and cooled to 0°C in an ice bath. Butyric acid (1.00 equiv., 1.0mol, 88.11g) was run in with stirring at such a rate as to prevent the temperature of the reaction form exceeding 40°C. After the addition of the last of the acid, the reaction was stirred on ice for 25 minutes. The resultant product was a pale yellow liquid with the following properties:
Viscosity @ 25°C: 5.7cP
Density @ 25°C: 0.92 g/cm³
Refractive Index @ 25°C: 1.440

### 31. N-Methylpiperidinium hexanoate

N-Methylpiperidine (1.0mol, 99.17g) was placed in an argon-flushed round-bottomed flask equipped with a magnetic stirrer and cooled to 0°C in an ice bath. Hexanoic acid (1.00 equiv., 1.0mol, 116.16g) was run in with stirring at such a rate as to prevent the temperature of the reaction form exceeding 40°C. After the addition of the last of the acid, the reaction was stirred on ice for 25 minutes. The resultant product was a pale yellow liquid with the following properties:
Viscosity @ 25°C: 6.8cP
Density @ 25°C: 0.90 g/cm³
Refractive Index @ 25°C: 1.443

### 32. N-Methylpiperidinium heptanoate

N-Methylpiperidine (1.0mol, 99.17g) was placed in an argon-flushed round-bottomed flask equipped with a magnetic stirrer and cooled to 0°C in an ice bath. Heptanoic acid (1.00 equiv., 1.0mol, 130.18g) was run in with stirring at such a rate as to prevent the temperature of the reaction form exceeding 40°C. After the addition of the last of the acid, the reaction was stirred on ice for 25 minutes. The resultant product was a pale yellow liquid with the following properties:
Viscosity @ 25°C: 7.7cP
Density @ 25°C: 0.90 g/cm³
Refractive Index @ 25°C: 1.444

### 33. N-Methylpiperidinium octanoate

N-Methylpiperidine (1.0mol, 99.17g) was placed in an argon-flushed round-bottomed flask equipped with a magnetic stirrer and cooled to 0°C in an ice bath. Octanoic acid (1.00 equiv., 1.0mol, 144.21g) was run in with stirring at such a rate as to prevent the temperature of the reaction form exceeding 40°C. After the addition of the last of the acid, the reaction was stirred on ice for 25 minutes. The resultant product was a pale yellow liquid with the following properties:
Density @ 25°C: 0.89 g/cm³
Refractive Index @ 25°C: 1.445

### 34. N,N,N-Triethylammonium propionate

Triethylamine (1.0mol, 101.19g) was placed in an argon-flushed round-bottomed flask equipped with a magnetic stirrer and cooled to 0°C in an ice bath. Propionic acid (1.00 equiv., 1.0mol, 74.08g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 3.5cP
Density @ 25°C: 0.88 g/cm³
Refractive Index @ 25°C: 1.423
π*:0.67

The product slowly oxidized on storage developing a pale brown colouration

### 35. N,N,N-Triethylammonium butyrate

Triethylamine (1.0mol, 101.19g) was placed in an argon-flushed round-bottomed flask equipped with a magnetic stirrer and cooled to 0°C in an ice bath. Butyric acid (1.00 equiv., 1.0mol, 88.11g) was run in with stirring at such a rate as to prevent the temperature of the reaction form exceeding 40°C. After the addition of the last of the acid, the reaction was stirred on ice for 25 minutes. The resultant product was a colourless liquid' with the following properties:
Viscosity @ 25°C: 3.9cP
Density @ 25°C: 0.86 g/cm³
Refractive Index @ 25°C: 1.423
π*: 0.66

The product slowly oxidized on storage developing a pale brown colouration

### 36. N,N,N-Triethylammonium hexanoate

Triethylamine (1.0mol, 101.19g) was placed in an argon-flushed round-bottomed flask equipped with a magnetic stirrer and cooled to 0°C in an ice bath. Hexanoic acid (1.00 equiv., 1.0mol, 116.16g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 4.9 cP
π*: 0.66

The product slowly oxidized on storage developing a pale brown colouration

### 37. N,N,N-Triethylammonium crotonate

Triethylamine (1.0mol, 101.19g) was placed in an argon-flushed round-bottomed flask equipped with a magnetic stirrer and cooled to 0°C in an ice bath. Crotonic acid (1.00 equiv., 1.0mol, 86.09g) was added portionwise with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 7.1cP
Density @ 25°C: 0.88 g/cm³
Refractive Index @ 25°C: 1.446
π*: 0.75

The product slowly oxidized on storage developing a pale brown colouration

### 38. N,N-Diisopropyl-N-ethylammonium butyrate

N,N-Diisopropylethylamine (1.0mol, 129.24g) was placed in an argon-flushed round-bottomed flask equipped with a magnetic stirrer and cooled to 0°C in an ice bath. Butyric acid (1.00 equiv., 1.0mol, 88.11g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 7.0cP
Density @ 25°C: 0.87 g/cm³
Refractive Index @ 25°C: 1.432
π*: 0.68
α: 1.18
β: 1.00
E_{N}^{T}: 0.81

### 39. N,N-Diisopropyl-N-ethylammonium octanoate

N,N-Diisopropylethylamine (1.0mol, 129.24g) was placed in an argon-flushed round-bottomed flask equipped with a magnetic stirrer and cooled to 0°C in an ice bath. Octanoic acid (1.00 equiv., 1.0mol, 144.21g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 11.8cP
Density @ 25°C: 0.86 g/cm³
Refractive Index @ 25°C: 1.439
E_{N}^{T}: 0.87

### 40. N,N,N-Trioctylammonium propionate

Trioctylamine (1.0mol, 353.67g) was placed in an argon-flushed round-bottomed flask equipped with a magnetic stirrer and cooled to 0°C in an ice bath. Propionic acid (1.00 equiv., 1.0mol, 74.08g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 17.0cP
Density @ 25°C: 0.84g/cm³
Refractive Index @ 25°C: 1.445
π*: 0.53

### 41. N,N,N-Trioctylammonium 2-oxobutyrate

Trioctylamine (1.0mol, 353.67g) was placed in an argon-flushed round-bottomed flask equipped with a magnetic stirrer and cooled to 0°C in an ice bath. 2-Ketobutyric acid (1.00 equiv., 1.0mol, 102.09g) was added portionwise with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 106.0cP
Density @ 25°C: 0.89g/cm³
Refractive Index @ 25°C: 1.457
π*: 0.80
E_{N}^{T}: 0.82

### 42. N,N,N-Trioctylammonium 3-toluate

Trioctylamine (1.0mol, 353.67g) was placed in an argon-flushed round-bottomed flask equipped with a magnetic stirrer and cooled to 0°C in an ice bath. m-Toluic acid (1.00 equiv., 1.0mol, 136.15g) was added portionwise with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 66.0cP
Density @ 25°C: 0.89g/cm³
Refractive Index @ 25°C: 1.478
π*: 0.69
E_{N}^{T}: 0.91

### 43. N,N,N-Triisobutylammonium butyrate

Triisobutylamine (1.0mol, 185.35g) was placed in an argon-flushed round-bottomed flask equipped with a magnetic stirrer and cooled to 0°C in an ice bath. Butyric acid (1.00 equiv., 1.0mol, 88.11g) was run in with stirring at such a rate as to prevent the temperature of the reaction form exceeding 40°C. After the addition of the last of the acid, the reaction was stirred on ice for 25 minutes. The resultant product was a colourless liquid with the following properties:
Viscosity @ 25°C: 3.3cP
Density @ 25°C: 0.82 g/cm³
Refractive Index @ 25°C: 1.418
π*: 0.46
β: 1.06

### Examples 44 to 163

The following compounds were also prepared and were all liquid at room temperature:
Trimethylammonium formate
Trimethylammonium acetate
Trimethylammonium propionate
Trimethylammonium butyrate
Trimethylammonium valerate
Trimethylammonium hexanoate
Trimethylammonium heptanoate
Trimethylaminonium octanoate
Trimethylammonium glycolate
N,N-Dimethylethylammonium formate
Dimethylethylammonium valerate
N,N-Dimethylethylammonium trans-2-hexenoate
N,N-Dimethylpropylammonium formate
N,N-Dimethylpropylammonium acetate
N,N-Dimethylpropylammonium propionate
N,N-Dimethylpropylammonium butyrate
N,N-Dimethylpropylammonium valerate
N,N-Dimethylpropylammonium hexanoate
N,N-Dimethylpropylammonium heptanoate
N,N-Dimethylpropylammonium octanoate
N,N-Dimethylbutylammonium formate
N,N-Dimethylhexylammonium valerate
N,N-Dimethyldecylammonium butyrate
N,N-Dimethyldecylammonium valerate
N,N-Dimethyldecylammonium hexanoate
N,N-Dirnethyldecylammonium heptanoate
N,N-Dimethyldecylammonium octanoate
N,N-Dimethyldecylammonium pivalate
N,N-Dimethyldodecylammonium valerate
N-Methyldiethylammonium acetate
N-Methyldiethylammonium propionate
N-Methyldiethylammonium butyrate
N-Methyldiethylammonium valerate
N-Methyldiethylammonium hexanoate
N-Methyldiethylammonium heptanoate
N-Methyldiethylammonium octanoate
N-Methyldipropylammonium acetate
N-Methyldipropylammonium propionate
N-Methyldipropylammonium butyrate
N-Methyldipropylammonium valerate
N-Methyldipropylammonium hexanoate
N-Methyldipropylammonium heptanoate
N-Methyldipropylammonium octanoate
N-Methyldibutylammonium acetate
N-Methyldibutylammonium propionate
N-Methyldibutylammonium butyrate
N-Methyldibutylammonium valerate
N-Methyldibutylammonium hexanoate
N-Methyldibutylammonium heptanoate
N-Methyldibutylammonium octanoate
N-Methyldicyclohexylammonium propionate
N-Methyldicyclohexylammonium butyrate
N-Methyldicyclohexylammonium valerate
N-Methyldieydohexylarnmonium hexanoate
N-Methyldicyclohexylammonium heptanoate
N-Methyldicyclohexylammonium octanoate
N-Ethyldiisopropylammonium formate
N-Ethyldiisopropylammonium valerate
N-Ethyldiisopropylammonium hexanoate
N-Ethyldiisopropylammonium heptanoate
N-Ethyldiisopropylammonium 2-methylvalerate
N-Ethyldiisopropylammonium 2-hexyldecanoate
Triethylammonium valerate
Triethylammonium heptanoate
Triethylammonium octanoate
Triethylammonium methanesulfonate
Tripropylammonium propionate
Tripropylammonium butyrate
Tripropylammonium valerate
Tripropylammonium hexanoate
Tripropylammonium heptanoate
Tripropylammonium octanoate
Tributylammonium propionate
Tributylammonium butyrate
Tributylammonium valerate
Tributylammonium hexanoate
Tributylammonium heptanoate
Tributylammonium octanoate
Triisobutylammonium valerate
Triisobutylammonium hexanoate
Triisobutylammonium heptanoate
Triisobutylammonium octanoate
Triisobutylammonium crotonate
Triisobutylammonium 2,2-dimethylbutyrate
Triisobutylammonium 2-methylvalerate
Triisobutylammonium decanoate
Trihexylammonium butyrate
Trihexylammonium valerate
Trihexylammonium hexanoate
Trihexylammonium heptanoate
Trihexylammonium octanoate
Trioctylammonium acetate
Trioctylammonium butyrate
Trioctylammonium valerate
Trioctylammonium hexanoate
Trioctylammonium heptanoate
Trioctylammonium octanoate
Trioctylammonium nonanoate
Trioctylammonium decanoate
Trioctylammonium crotonate
Trioctylammonium trans-2-hexenoate
Trioctylammonium 2-butyloctanoate
Trioctylammonium benzoate
Trioctylammonium phenylacetate
Triisooctylammonium propionate
Triisooctylammonium butyrate
Triisooctylammonium valerate
Triisooctylammonium hexanoate
Triisooctylammonium heptanoate
Triisooctylammonium octanoate
N-Methylpiperidinium formate
N-Methylpiperidinium valerate
N-Methylpyrrolidinium formate
N-Methylpyrrolidinium acetate
N-Methylpyrrolidinium propionate
N-Methylpyrrolidinium butyrate
N-Methylpyrrolidinium valerate
N-Methylpyrrolidinium hexanoate
N-Methylpyrrolidinium heptanoate
N-Methylpyrrolidinium octanoate

### Example 164

### EXTRACTION OF ARTEMISININ USING TRIETHYLAMMONIUM PROPIONATE AS A SOLVENT

Dried *Artemisia annua* aerial parts (1kg) were placed into a stirred extraction vessel containing 3L triethylammonium propionate and stirred for 45 minutes at room temperature. At the completion of this period, the plant material and insoluble residues were removed by filtration (yielding 2.1L of primary extract) and the extract was analysed by High Performance Liquid Chromatography, utilizing Refractive Index detection to quantify the level of artemisinin present. This was determined to equate to 0.95% by mass of the dry biomass. Four minor impurities were also detected.

Distilled water was then added to the ionic liquid primary extract in the ratio of 3 parts water to 4 parts IL, with external cooling and stirring, over a period of 15 minutes. This elicited the precipitation of a brown gelatinous material, containing 97% of the theoretical amount of artemisinin present in the primary extract. This gelatinous material was dissolved in ethyl acetate, chromatographed on a short silica column and the artemisinin containing fractions were pooled, stripped of solvent *in vacuo* and recrystallised to yield artemisinin in 97% yield and 995 purity by mass spectrometry and HPLC. No residual triethylammonium propionate could be detected in the artemisinin using either ion chromatography or HPLC.

## Claims

1. The use of a trialkylammonium salt as a protic ionic liquid wherein the trialkylammonium moiety is selected from the group consisting of N,N-dimethylethylammonium acetate; N,N- dimethylethylammonium propionate; N,N-dimethylethylammonium butyrate; N,N- dimethylethylammonium hexanoate; N,N-dimethylethylammonium heptanoate; N,N- dimethylethylammonium octanoate; N,N-dimethylethylammonium crotonate; N,N- dimethylethylammonium phenylacetate; N,N-dimethylethylammonium 2,2- dimethylbutyrate; N,N-dimethylbutylammonium acetate; N,N- dimethylbutylammonium propionate; N,N-dimethylbutylammonium butyrate; N,N- dimethylbutylammonium hexanoate; N,N-dimethylbutylammonium heptanoate; N,N- dimethylbutylammonium octanoate; N,N-dimethylbutylammonium benzoate; N,N- dimethylbutylammonium phenylacetate; N,N-dimethylbutylammonium 2,2- dimethylbutyrate; N,N-dimethylbutylammonium 2-hexyldecanoate; N,N- dimethylbutylammonium 2-methylvalerate; N,N-dimethylbutylammonium decanoate; N,N-dimethylhexylammonium acetate; N,N-dimethyldodecylammonium propionate; N,N-dimethyldodecylammonium butyrate; N,N-dimethyldodecylammonium hexanoate; N,N-dimethyldodecylammonium heptanoate; N,N- dimethyldodecylammonium octanoate; N-methylpiperidinium acetate; N- methylpiperidinium propionate; N-methylpiperidinium butyrate; N-methylpiperidinium hexanoate; N-methylpiperidinium heptanoate; N,N,N-triethylammonium propionate; N,N,N-triethylammonium butyrate; N,N,N-triethylammonium hexanoate; N,N,N-triethylammonium crotonate; N,N-diisopropyl-N-ethylammonium butyrate; N,N-diisopropyl-N-ethylammonium octanoate; N,N,N-trioctylammonium propionate; N,N,N-trioctylammonium 2-oxobutyrate; N,N,N-trioctylammonium 3-toluate; N,N,N-triisobutylammonium butyrate; trimethylammonium formate; trimethylammonium acetate; trimethylammonium propionate; trimethylammonium butyrate; trimethylammonium valerate; trimethylammonium hexanoate; trimethylammonium heptanoate; trimethylammonium octanoate; trimethylammonium glycolate; N,N-dimethylethylammonium formate; N,N-dimethylethylammonium valerate; N,N-dimethylethylammonium trans-2-hexenoate; N,N-dimethylpropylammonium formate; N,N-dimethylpropylammonium acetate; N,N-dimethylpropylammonium propionate; N,N-dimethylpropylammonium butyrate; N,N-dimethylpropylammonium valerate; N,N-dimethylpropylammonium hexanoate; N,N-dimethylpropylammonium heptanoate; N,N-dimethylpropylammonium octanoate; N,N-dimethylbutylammonium formate; N,N-dimethylhexylammonium valerate; N,N-dimethyldecylammonium butyrate; N,N-dimethyldecylammonium valerate; N,N-dimethyldecylammonium hexanoate; N,N-dimethyldecylammonium heptanoate; N,N-dimethyldecylammonium octanoate; N,N-dimethyldecylammonium pivalate; N,N-dimethyldodecylammonium valerate; N-methyldiethylammonium acetate; N-methyldiethylammonium propionate; N-methyldiethylammonium butyrate; N-methyldiethylammonium valerate; N-methyldiethylammonium hexanoate; N-methyldiethylammonium heptanoate; N-methyldiethylammonium octanoate; N-methyldipropylammonium acetate; N-methyldipropylammonium propionate; N-methyldipropylammonium butyrate; N-methyldipropylammonium valerate; N-methyldipropylammonium hexanoate; N-methyldipropylammonium heptanoate; N-methyldipropylammonium octanoate; N-methyldibutylammonium acetate; N-methyldibutylammonium propionate; N-methyldibutylammonium butyrate; N-methyldibutylammonium valerate; N-methyldibutylammonium hexanoate; N-methyldibutylammonium heptanoate; N-methyldibutylammonium octanoate; N-methyldicyclohexylammonium propionate; N-methyldicyclohexylammonium butyrate; N-methyldicyclohexylammonium valerate; N-methyldicyclohexylammonium hexanoate; N-methyldicyclohexylammonium heptanoate; N-methyldicyclohexylammonium octanoate; N-ethyldiisopropylammonium formate; N-ethyldiisopropylammonium valerate; N-ethyldiisopropylammonium hexanoate; N-ethyldiisopropylammonium heptanoate; N-ethyldiisopropylammonium 2-methylvalerate; N-ethyldiisopropylammonium 2-hexyldecanoate; triethylammonium valerate; tlethylammonium heptanoate; triethylammonium octanoate; triethylammonium methanesulfonate; tripropylammonium propionate; tripropylammonium butyrate; tripropylammonium valerate; tripropylammonium hexanoate; tripropylammonium heptanoate; tripropylammonium octanoate; tributylammonium propionate; tributylammonium butyrate; tributylammonium valerate; tributylammonium hexanoate; tributylammonium heptanoate; tributylammonium octanoate; triisobutylammonium valerate; triisobutylammonium hexanoate; triisobutylammonium heptanoate; triisobutylammonium octanoate; triisobutylammonium crotonate; triisobutylammonium 2,2-dimethylbutyrate; triisobutylammonium 2-methylvalerate; triisobutylammonium decanoate; trihexylammonium butyrate; trihexylammonium valerate; trihexylammonium hexanoate; trihexylammonium heptanoate; trihexylammonium octanoate; trioctylammonium acetate; trioctylammonium butyrate; trioctylammonium valerate; trioctylammonium hexanoate; trioctylammonium heptanoate; trioctylammonium octanoate; trioctylammonium nonanoate; trioctylammonium decanoate; trioctylammonium crotonate; trioctylammonium trans-2-hexenoate; trioctylammonium 2-butyloctanoate; trioctylammonium benzoate; trioctylammonium phenylacetate; triisooctylammonium propionate; triisooctylammonium butyrate; triisooctylammonium valerate; triisooctylammonium hexanoate; triisooctylammonium heptanoate; triisooctylammonium octanoate; N-methylpiperidinium formate; N-methylpiperidinium valerate; N-methylpyrrolidinium formate; N-methylpyrrolidinium acetate; N-methylpyrrolidinium propionate; N-methylpyrrolidinium butyrate; N-methylpyrrolidinium valerate; N-methylpyrrolidinium hexanoate; N-methylpyrrolidinium heptanoate; and N-methylpyrrolidinium octanoate.

2. The use according to claim 1 wherein the trialkylammonium moiety is triethylammonium.

3. The use according to claim 2 wherein the trialkylammonium salt is triethylammonium propionate.

4. The use according to claim 1 wherein the solvent is an extraction solvent.

5. The use according to claim 4 wherein the solvent is used in the extraction of a natural product.

6. The use according to claim 5 wherein the natural product is a terpene.

7. A method of manufacturing an organic compound which comprises the use of a protic ionic liquid comprising a trialkylammonium salt as a solvent wherein the trialkylammonium moiety is selected from the group consisting of N,N-dimethylethylammonium acetate; N,N- dimethylethylammonium propionate; N,N-dimethylethylammonium butyrate; N,N- dimethylethylammonium hexanoate; N,N-dimethylethylammonium heptanoate; N,N- dimethylethylammonium octanoate; N,N-dimethylethylammonium crotonate; N,N- dimethylethylammonium phenylacetate; N,N-dimethylethylammonium 2,2- dimethylbutyrate; N,N-dimethylbutylammonium acetate; N,N- dimethylbutylammonium propionate; N,N-dimethylbutylammonium butyrate; N,N- dimethylbutylammonium hexanoate; N,N-dimethylbutylammonium heptanoate; N,N- dimethylbutylammonium octanoate; N,N-dimethylbutylammonium benzoate; N,N- dimethylbutylammonium phenylacetate; N,N-dimethylbutylammonium 2,2- dimethylbutyrate; N,N-dimethylbutylammonium 2-hexyldecanoate; N,N- dimethylbutylammonium 2-methylvalerate; N,N-dimethylbutylammonium decanoate; N,N-dimethylhexylammonium acetate; N,N-dimethyldodecylammonium propionate; N,N-dimethyldodecy lammonium butyrate; N,N-dimethyldodecylammonium hexanoate; N,N-dimethyldodecylammonium heptanoate; N,N- dimethyldodecylammonium octanoate; N-methylpiperidinium acetate; N-methylpiperidinium propionate; N-methylpiperidinium butyrate; N-methylpiperidinium hexanoate; N-methylpiperidinium heptanoate; N,N,N-triethylammonium propionate; N,N,N-triethylammonium butyrate; N,N,N-triethylammonium hexanoate; N,N,N-triethylammonium crotonate; N,N-diisopropyl-N-ethylammonium butyrate; N,N-diisopropyl-N-ethylammonium octanoate; N,N,N-trioctylammonium propionate; N,N,N-trioctylammonium 2-oxobutyrate; N,N,N-trioctylammonium 3-toluate; N,N,N-triisobutylarrimonium butyrate; trimethylammonium formate; trimethylammonium acetate; trimethylammonium propionate; trimethylammonium butyrate; trimethylammonium valerate; trimethylammonium hexanoate; trimethylammonium heptanoate; trimethylammonium octanoate; trimethylammonium glycolate; N,N-dimethylethylammonium formate; N,N-dimethylethylammonium valerate; N,N-dimethylethylammonium trans-2-hexenoate; N,N-dimethylpropylammonium formate; N,N-dimethylpropylammonium acetate; N,N-dimethylpropylammonium propionate; N,N-dimethylpropylammonium butyrate; N,N-dimethylpropylammonium valerate; N,N-dimethylpropylammonium hexanoate; N,N-dimethylpropylammonium heptanoate; N,N-dimethylpropylammonium octanoate; N,N-dimethylbutylammonium formate; N,N-dimethylhexylammonium valerate; N,N-dimethyldecylammonium butyrate; N,N-dimethyldecylammonium valerate; N,N-dimethyldecylammonium hexanoate; N,N-dimethyldecylammonium heptanoate; N,N-dimethyldecylammonium octanoate; N,N-dimethyldecylammonium pivalate; N,N-dimethyldodecylammonium valerate; N-methyldiethylammonium acetate; N-methyldiethylammonium propionate; N-methyldiethylammonium butyrate; N-methyldiethylammonium valerate; N-methyldiethylammonium hexanoate; N-methyldiethylammonium heptanoate; N-methyldiethylammonium octanoate; N-methyldipropylammonium acetate; N-methyldipropylammonium propionate; N-methyldipropylammonium butyrate; N-methyldipropylammonium valerate; N-methyldipropylammonium hexanoate; N-methyldipropylammonium heptanoate; N-methyldipropylammonium octanoate; N-methyldibutylammonium acetate; N-methyldibutylammonium propionate; N-methyldibutylammonium butyrate; N-methyldibutylammonium valerate; N-methyldibutylammonium hexanoate; N-methyldibutylammonium heptanoate; N-methyldibutylammonium octanoate; N-methyldicyclohexylammonium propionate; N-methyldicyclohexylammonium butyrate; N-methyldicyclohexylammonium valerate; N-methyldicyclohexylammonium hexanoate; N-methyldicyclohexylammonium heptanoate; N-methyldicyclohexylammonium octanoate; N-Ethyldiisopropylammonium formate; N-Ethyldiisopropylammonium valerate; N-Ethyldiisopropylammonium hexanoate; N-Ethyldiisopropylammonium heptanoate; N-Ethyldiisopropylammonium 2-methylvalerate; N-Ethyldiisopropylammonium 2-hexyldecanoate; triethylammonium valerate; triethylammonium heptanoate; triethylammonium octanoate; triethylammonium methanesulfonate; tripropylammonium propionate; tripropylammonium butyrate; tripropylammonium valerate; tripropylammonium hexanoate; tripropylammonium heptanoate; tripropylammonium octanoate; tributylammonium propionate; tributylammonium butyrate; tributylammonium valerate; tributylammonium hexanoate; tributylammonium heptanoate; tributylammonium octanoate; triisobutylammonium valerate; triisobutylammonium hexanoate; triisobutylammonium heptanoate; triisobutylammonium octanoate; triisobutylammonium crotonate; triisobutylammonium 2,2-dimethylbutyrate; triisobutylammonium 2-methylvalerate; triisobutylammonium decanoate; trihexylammonium butyrate; trihexylammonium valerate; trihexylammonium hexanoate; trihexylammonium heptanoate; trihexylammonium octanoate; trioctyl ammonium acetate; trioctylammonium butyrate; trioctylammonium valerate; trioctylammonium hexanoate; trioctylammonium heptanoate; trioctylammonium octanoate; trioctylammonium nonanoate; trioctylammonium decanoate; trioctylammonium crotonate; trioctylammonium trans- 2-hexenoate; trioctylammonium 2-butyloctanoate; trioctylammonium benzoate; trioctylammonium phenylacetate; triisooctylammonium propionate; triisooctylammonium butyrate; triisooctylammonium valerate; triisooctylammonium hexanoate; triisooctylammonium heptanoate; triisooctylammonium octanoate; N- methylpiperidinium formate; N-methylpiperidinium valerate; N-methylpyrrolidinium formate; N-methylpyrrolidinium acetate; N-methylpyrrolidinium propionate; N- methylpyrrolidinium butyrate; N-methylpyrrolidinium valerate; N- methylpyrrolidinium hexanoate; N-methylpyrrolidinium heptanoate; and N- methylpyrrolidinium octanoate.

8. A method according to claim 7 wherein the trialkylammonium moiety is triethylammonium.

9. A method according to claim 7 wherein the anion of the salt is an organic acid and the organic acid is a carboxylic acid.

10. A method according to claim 9 wherein the organic acid is a Cl to 10 carboxylic acid

11. A method according to claim 10 wherein the salt of the organic acid is selected from the group consisting of formate, acetate, propionate and butyrate.

12. A method according to claim 8 wherein the trialkylammonium salt is triethylammonium propionate.

13. A method according to claim 7 wherein the method comprises extraction of a natural product.

14. A method according to claim 13 wherein the natural product is a terpene.

## Patentansprüche

1. Die Verwendung und Benutzung von Trialkylammoniumsalz, als eine protisch ionische Flüssigkeit, wobei der Trialkylammoniumanteil aus der Gruppe ausgewählt wurde die N,N- Dimethyläthylammonium acetat; N,N- Dimethyläthylammonium propionat; N,N-Dimethyläthylammonium butyrat; N, N- Dimethyläthylammonium hexanoat; N,N-Dimethyläthylammonium heptanoat; N, N- Dimethyläthylammonium octanoat; N, N-Dimethyläthylammonium crotonat; N, N- Dimethyläthylammonium phenylacetat; N, N-Dimethyläthylammonium 2,2-dimethylbutyrat; N,N-Dimethylbutylammoniumacetat; N,N-Dimethylbutylammonium propionat; N,N-Dimethylbutylammoniumbutyrat; N,N-Dimethylbutylammonium hexanoat; N,N-Dimethylbutylammonium heptanoat; N,N-Dimethylbutylammonium octanoat; N,N-Dimethylbutylammonium benzoat; N,N-Dimethylbutylammonium phenylacetat; N,N-Dimethylbutylammonium 2,2- dimethylbutyrat; N,N-Dimethylbutylammonium 2-hexyldecanoat; N,N-Dimethylbutylammonium 2-methylvalerat; N,N-Dimethylbutylammonium decanoat; N,N-Dimethylhexylammonium acetat; N,N-Dimethyldodecylammonium propionat; N,N-Dimethyldodecylammonium butyrat; N,N-Dimethyldodecylammoniumhexanoat; N,N-Dimethyldodecylammonium heptanoat; N,N-Dimethyldodecylammonium octanoat; N-Methylpiperidinium acetat; N-Methylpiperidinium propionat; ; N-Methylpiperidinium butyrat; ; N-Methylpiperidinium hexanoat; ; N-Methylpiperidinium heptanoat; N,N,N-Triäthylammonium propionat; N,N,N-Triäthylammonium butyrat; N,N,N-Triäthylammonium hexanoat; N,N,N-Triäthylammonium crotonat; N-Äthyl-N,N-diisopropylammonium butyrat; N-Äthyl-N,N-diisopropylammonium octanoat; N,N,N-Trioctylammonium 2-oxobutyrat; N,N,N-Trioctylammonium 3-toluat; N,N,N-Triisobutylammonium butyrat; N,N,N-Trimethylammonium formiat; N,N,N-Trimethylammonium acetat; N,N,N-Trimethylammonium propionat; N,N,N-Trimethylammonium butyrat; N,N,N-Trimethylammonium valerat; N,N,N-Trimethylammonium hexanoat; N,N,N-Trimethylammonium heptanoat; N,N,N-Trimethylammonium octanoat; N,N,N-Trimethylammonium glycolat; N,N-Dimethyläthylammonium formiat; N,N- Dimethyläthylammonium valerat; N,N-Dimethyläthylammonium trans-2-hexenoat; N,N-Dimethylpropylammonium formiat; N,N-Dimethylpropylammonium acetat; N,N-Dimethylpropylammonium propionat; N,N-Dimethylpropylammonium butyrat; N,N-Dimethylpropylammonium valerat; N,N-Dimethylpropylammonium hexanoat; N,N-Dimethylpropylammonium heptanoat; N,N-Dimethylpropylammonium octanoat; N,N-Dimethylbutylammonium formiat; N,N-Dimethylhexylammonium valerat; N,N-Dimethyldecylammonium butyrat; N,N-Dimethyldecylammonium valerat; N,N-Dimethyldecylammoniumhexanoat; N,N-Dimethyldecylammonium heptanoat; N,N-Dimethyldecylammonium octanoat; N,N-Dimethyldecylammonium pivalat; N,N-Dimethyldodecylammonium valerat; N-Methyldiäthylammonium acetat; N-Methyldiäthylammonium propionat; N-Methyldiäthylammonium butyrat; N-Methyldiäthylammonium valerat; N-Methyldiäthylammonium hexanoat; N-Methyldiäthylammonium heptanoat; N-Methyldiäthylammonium octanoat; N-Methyldipropylammonium acetat; N-Methyldipropylammonium propionat; N-Methyldipropylammonium butyrat; N-Methyldipropylammonium valerat; N-Methyldipropylammonium hexanoat; N-Methyldipropylammonium heptanoat; N-Methyldipropylammonium octanoat; N-Methyldibutylammonium acetat; N-Methyldibutylammonium propionat; N-Methyldibutylammonium butyrat; N-Methyldibutylammonium valerat; N-Methyldibutylammonium hexanoat; N-Methyldibutylammonium heptanoat; N-Methyldibutylammonium octanoat; N-Methyldicyclohexylammonium propionat; N-Methyldicyclohexylammonium butyrat; N-Methyldicyclohexylammonium valerat; N-Methyldicyclohexylammonium hexanoat; N-Methyldicyclohexylammonium heptanoat; N-Methyldicyclohexylammonium octanoat; N-Äthyl-N,N-diisopropylammonium formiat; N-Äthyl-N,N-diisopropylamjmonium valerat; N-Äthyl-N,N-diisopropylammonium hexanoat; N-Äthyl-N,N-diisopropylammonium heptanoat; N-Äthyl-N,N-diisopropylammonium 2-methylvalerat; N-Äthyl-N,N-diisopropylammonium 2-hexyldecanoat; N,N,N-Triäthylammonium valerat; N,N,N-Triäthylammonium heptanoat; N,N,N-Triäthylammonium octanoat; N,N,N-Triäthylammonium methylsulfonat; N,N,N-Tripropylammonium propionat; N,N,N-Tripropylammonium butyrat; N,N,N-Tripropylammonium valerat; N,N,N-Tripropylammonium hexanoat; N,N,N-Tripropylammonium heptanoat; N,N,N-Tripropylammonium octanoat; N,N,N-Tributylammonium propionat; N,N,N-Tributylammonium butyrat; N,N,N-Tributylammonium valerat; N,N,N-Tributylammonium hexanoat; N,N,N-Tributylammonium heptanoat; N,N,N-Tributylammonium octanoat; N,N,N-Triisobutylammonium valerat; N,N,N-Triisobutylammonium hexanoat; N,N,N-Triisobutylammonium heptanoat; N,N,N-Triisobutylammonium octanoat; N,N,N-Triisobutylammonium crotonat; N,N,N-Triisobutylammonium 2,2-dimethylbutyrat; N,N,N-Triisobutylammonium 2-methylvalerat; N,N,N-Triisobutylammonium decanoat; N,N,N-Trihexylammonium butyrat; N,N,N-Trihexylammonium valerat; N,N,N-Trihexylammonium hexanoat; N,N,N-Trihexylammonium heptanoat; N,N,N-Trihexylammonium octanoat; N,N,N-Trioctylammonium acetat; N,N,N-Trioctylammonium butyrat; N,N,N-Trioctylammonium valerat; N,N,N-Trioctylammonium hexanoat; N,N,N-Trioctylammonium heptanoat; N,N,N-Trioctylammonium octanoat; N,N,N-Trioctylammonium nonanoat; N,N,N-Trioctylammonium decanoat; N,N,N-Trioctylammonium crotonat; N,N,N-Trioctylammonium trans-2-hexenoat; N,N,N-Trioctylammonium 2-butyloctanoat; N,N,N-Trioctylammonium benzoat; N,N,N-Trioctylammonium phenylacetat; N,N,N-Triisooctylammonium propionat; N,N,N-Triisooctylammonium butyrat; N,N,N-Triisooctylammonium valerat; N,N,N-Triisooctylammonium hexanoat; N,N,N-Triisooctylammonium heptanoat; N,N,N-Triisooctylammonium octanoat; N-Methylpiperidinium formiat; N-Methylpiperidinium N-Methylpiperidinium valerat; N-Methylpyrrolidinium formiat; N-Methylpyrrolidinium acetat; N-Methylpyrrolidinium propionat; N-Methylpyrrolidinium butyrat; N-Methylpyrrolidinium valerat; N-Methylpyrrolidinium hexanoat; N-Methylpyrrolidinium heptanoat und N-Methylpyrrolidinium octanoat enthalten.

2. Die Verwendung und Benutzung nach Anspruch 1, wobei der Trialkylammoniumanteil aus Triäthylammonium besteht.

3. Die Verwendung und Benutzung nach Anspruch 2, wobei das Trialkylammoniumsalz aus Triäthylammoniumpropionate besteht.

4. Die Verwendung und Benutzung nach Anspruch 1, wobei das Lösungsmittel eine Lösungsmittel-Extraktion ist.

5. Die Verwendung und Benutzung nach Anspruch 4, wobei das Lösungsmittel als Extraktion für ein natürliches Produkt verwendet wird.

6. Die Verwendung und Benutzung nach Anspruch 5, wobei das natürliche Produkt ein Terpen ist.

7. Eine Methode zur Herstellung einer organischen Verbindung welche den Gebrauch von protisch ionischer Flüssigkeit enthält, welche Trialkylammoniumsalz als Lösungsmittel enthält. Der Trialkylammoniumanteil wurde aus einer Gruppe ausgewählt, die N,N-Dimethyläthylammonium acetat; N,N- Dimethyläthylammonium propionat; N,N-Dimethyläthylammonium butyrat; N, N- Dimethyläthylammonium hexanoat; N,N-Dimethyläthylammonium heptanoat; N, N- Dimethyläthylammonium octanoat; N, N-Dimethyläthylammonium crotonat; N, N- Dimethyläthylammonium phenylacetat; N, N-Dimethyläthylammonium 2,2-dimethylbutyrat; N,N-Dimethylbutylammoniumacetat; N,N-Dimethylbutylammonium propionat; N,N-Dimethylbutylammoniumbutyrat; N,N-Dimethylbutylammonium hexanoat; N,N-Dimethylbutylammonium heptanoat; N,N-Dimethylbutylammonium octanoat; N,N-Dimethylbutylammonium benzoat; N,N-Dimethylbutylammonium phenylacetat; N,N-Dimethylbutylammonium 2,2- dimethylbutyrat; N,N-Dimethylbutylammonium 2-hexyldecanoat; N,N-Dimethylbutylammonium 2-methylvalerat; N,N-Dimethylbutylammonium decanoat; N,N-Dimethylhexylammonium acetat; N,N-Dimethyldodecylammonium propionat; N,N-Dimethyldodecylammonium butyrat; N,N-Dimethyldodecylammoniumhexanoat; N,N-Dimethyldodecylammonium heptanoat; N,N-Dimethyldodecylammonium octanoat; N-Methylpiperidinium acetat; N-Methylpiperidinium propionat; ; N-Methylpiperidinium butyrat; ; N-Methylpiperidinium hexanoat; ; N-Methylpiperidinium heptanoat; N,N,N-Triäthylammonium propionat; N,N,N-Triäthylammonium butyrat; N,N,N-Triäthylammonium hexanoat; N,N,N-Triäthylammonium crotonat; N-Äthyl-N,N-diisopropylammonium butyrat; N-Äthyl-N,N-diisopropylammonium octanoat; N,N,N-Trioctylammonium 2-oxobutyrat; N,N,N-Trioctylammonium 3-toluat; N,N,N-Triisobutylammonium butyrat; N,N,N-Trimethylammonium formiat; N,N,N-Trimethylammonium acetat; N,N,N-Trimethylammonium propionat; N,N,N-Trimethylammonium butyrat; N,N,N-Trimethylammonium valerat; N,N,N-Trimethylammonium hexanoat; N,N,N-Trimethylammonium heptanoat; N,N,N-Trimethylammonium octanoat; N,N,N-Trimethylammonium glycolat; N,N-Dimethyläthylammonium formiat; N,N- Dimethyläthylammonium valerat; N,N-Dimethyläthylammonium trans-2-hexenoat; N,N-Dimethylpropylammonium formiat; N,N-Dimethylpropylammonium acetat; N,N-Dimethylpropylammonium propionat; N,N-Dimethylpropylammonium butyrat; N,N-Dimethylpropylammonium valerat; N,N-Dimethylpropylammonium hexanoat; N,N-Dimethylpropylammonium heptanoat; N,N-Dimethylpropylammonium octanoat; N,N-Dimethylbutylammonium formiat; N,N-Dimethylhexylammonium valerat; N,N-Dimethyldecylammonium butyrat; N,N-Dimethyldecylammonium valerat; N,N-Dimethyldecylammoniumhexanoat; N,N-Dimethyldecylammonium heptanoat; N,N-Dimethyldecylammonium octanoat; N,N-Dimethyldecylammonium pivalat; N,N-Dimethyldodecylammonium valerat; N-Methyldiäthylammonium acetat; N-Methyldiäthylammonium propionat; N-Methyldiäthylammonium butyrat; N-Methyldiäthylammonium valerat; N-Methyldiäthylammonium hexanoat; N-Methyldiäthylammonium heptanoat; N-Methyldiäthylammonium octanoat; N-Methyldipropylammonium acetat; N-Methyldipropylammonium propionat; N-Methyldipropylammonium butyrat; N-Methyldipropylammonium valerat; N-Methyldipropylammonium hexanoat; N-Methyldipropylammonium heptanoat; N-Methyldipropylammonium octanoat; N-Methyldibutylammonium acetat; N-Methyldibutylammonium propionat; N-Methyldibutylammonium butyrat; N-Methyldibutylammonium valerat; N-Methyldibutylammonium hexanoat; N-Methyldibutylammonium heptanoat; N-Methyldibutylammonium octanoat; N-Methyldicyclohexylammonium propionat; N-Methyldicyclohexylammonium butyrat; N-Methyldicyclohexylammonium valerat; N-Methyldicyclohexylammonium hexanoat; N-Methyldicyclohexylammonium heptanoat; N-Methyldicyclohexylammonium octanoat; N-Äthyl-N,N-diisopropylammonium formiat; N-Äthyl-N,N-diisopropylammonium valerat; N-Äthyl-N,N-diisopropylammonium hexanoat; N-Äthyl-N,N-diisopropylammonium heptanoat; N-Äthyl-N,N-diisopropylammonium 2-methylvalerat; N-Äthyl-N,N-diisopropylammonium 2-hexyldecanoat; N,N,N-Triäthylammonium valerat; N,N,N-Triäthylammonium heptanoat; N,N,N-Triäthylammonium octanoat; N,N,N-Triäthylammonium methylsulfonat; N,N,N-Tripropylammonium propionat; N,N,N-Tripropylammonium butyrat; N,N,N-Tripropylammonium valerat; N,N,N-Tripropylammonium hexanoat; N,N,N-Tripropylammonium heptanoat; N,N,N-Tripropylammonium octanoat; N,N,N-Tributylammonium propionat; N,N,N-Tributylammonium butyrat; N,N,N-Tributylammonium valerat; N,N,N-Tributylammonium hexanoat; N,N,N-Tributylammonium heptanoat; N,N,N-Tributylammonium octanoat; N,N,N-Triisobutylammonium valerat; N,N,N-Triisobutylammonium hexanoat; N,N,N-Triisobutylammonium heptanoat; N,N,N-Triisobutylammonium octanoat; N,N,N-Triisobutylammonium crotonat; N,N,N-Triisobutylammonium 2,2-dimethylbutyrat; N,N,N-Triisobutylammonium 2-methylvalerat; N,N,N-Triisobutylammonium decanoat; N,N,N-Trihexylammonium butyrat; N,N,N-Trihexylammonium valerat; N,N,N-Trihexylammonium hexanoat; N,N,N-Trihexylammonium heptanoat; N,N,N-Trihexylammonium octanoat; N,N,N-Trioctylammonium acetat; N,N,N-Trioctylammonium butyrat; N,N,N-Trioctylammonium valerat; N,N,N-Trioctylammonium hexanoat; N,N,N-Trioctylammonium heptanoat; N,N,N-Trioctylammonium octanoat; N,N,N-Trioctylammonium nonanoat; N,N,N-Trioctylammonium decanoat; N,N,N-Trioctylammonium crotonat; N,N,N-Trioctylammonium trans-2-hexenoat; N,N,N-Trioctylammonium 2-butyloctanoat; N,N,N-Trioctylammonium benzoat; N,N,N-Trioctylammonium phenylacetat; N,N,N-Triisooctylammonium propionat; N,N,N-Triisooctylammonium butyrat; N,N,N-Triisooctylammonium valerat; N,N,N-Triisooctylammonium hexanoat; N,N,N-Triisooctylammonium heptanoat; N,N,N-Triisooctylammonium octanoat; N-Methylpiperidinium formiat; N-Methylpiperidinium N-Methylpiperidinium valerat; N-Methylpyrrolidinium formiat; N-Methylpyrrolidinium acetat; N-Methylpyrrolidinium propionat; N-Methylpyrrolidinium butyrat; N-Methylpyrrolidinium valerat; N-Methylpyrrolidinium hexanoat; N-Methylpyrrolidinium heptanoat und N-Methylpyrrolidinium octanoat enthält.

8. Ein Verfahren nach Anspruch 7, wobei der Trialkylammoniumanteil aus Trialkylammonium besteht.

9. Ein Verfahren nach Anspruch 7, wobei die Anionen eines Salzes eine organische Säure und die organische Säure eine Carbonsäure ist.

10. Ein Verfahren nach Anspruch 9, wobei die organische Säure ein C1 zu 10 Carbonsäuren ist.

11. Ein Verfahren nach Anspruch 10, wobei das Salz der organischen Säure aus der Gruppe ausgewählt wurde, die aus Formiaten, Acetaten, Propionaten und Butyraten besteht.

12. Ein Verfahren nach Anspruch 8, wobei das Trialkylammoniumsalz aus Triäthylammoniumpropionaten besteht.

13. Ein Verfahren nach Anspruch 7, wobei diese Methode eine Extraktion von einem natürlichen Produkt enthält.

14. Ein Verfahren nach Anspruch 13, wobei das natürliche Produkt ein Terpen ist.

## Revendications

1. Utilisation du sel trialkylammonium comme liquide ionique protique dans laquelle un groupement trialkylammonium est sélectionné à partir de groupes composés d'acétate de N,N-diméthyléthylammonium; propanoate de N,N-diméthyléthylammonium; butanoate de N,N-diméthyléthylammonium; hexanoate de N,N-diméthyléthylammonium; heptanoate de N,N-diméthyléthylammonium; octanoate de N,N-diméthyléthylammonium; crotonate de N,N-diméthyléthylammonium; phenylacétate de N,N-diméthyléthylammonium; 2,2-diméthylbutanoate de N,N-diméthyléthylammonium; acétate de N,N-diméthylbutylammonium; propanoate de N,N-diméthylbutylammonium; butanoate de N,N-diméthylbutylammonium; hexanoate de N,N-diméthylbutylammonium; heptanoate de N,N-diméthylbutylammonium; octanoate de N,N-diméthylbutylammonium; benzoate de N,N-diméthylbutylammonium; phenylacétate de N,N-diméthylbutylammonium; 2,2-diméthylbutanoate de N,N-diméthylbutylammonium; 2-hexyldécanoate de N,N-diméthylbutylammonium; 2-méthylvalérate de N,N-diméthylbutylammonium; décanoate de N,N-diméthylbutylammonium; acétate de N,N-diméthylhexylammonium; propanoate de diméthyldodécylammonium; butanoate de N,N-diméthyldodécylammonium; hexanoate de N,N-diméthyldodécylammonium; heptanoate de N,N-diméthyldodécylammonium; octanoate de N,N-diméthyldodécylammonium; acétate de N-méthylpipéridinium; propanoate de N-méthylpipéridinium; butanoate de N-méthylpipéridinium; hexanoate de N-méthylpipéridinium; heptanoate de N-méthylpipéridinium; propanoate de N,N,N-triéthylammonium; butanoate de N,N,N-triéthylammonium; hexanoate de N,N,N-triéthylammonium; crotonate de N,N,N-triéthylammonium; butanoate de N,N-diisopropyl-N-éthylammonium; octanoate de N,N-diisopropyl-N-éthylammonium; propanoate de N,N,N-trioctylammonium; 2-oxobutanoate de N,N,N-trioctylammonium; 3-toluate de N,N,N-trioctylammonium; butanoate de N,N,N-triisobutylammonium; formiate de triméthylammonium; acétate de triméthylammonium; propanoate de triméthylammonium; butanoate de triméthylammonium; valérate de triméthylammonium; hexanoate de triméthylammonium; heptanoate de triméthylammonium; octanoate de triméthylammonium; glycolate de triméthylammonium; formiate de N,N-diméthyléthylammonium; valérate de N,N-diméthyléthylammonium; trans-2-hexénoate de N,N-diméthyléthylammonium; formiate de N,N-diméthylpropylammonium; acétate de N,N-diméthylpropylammonium; propanoate de N,N-diméthylpropylammonium; butanoate de N,N-diméthylpropylammonium; valérate de N,N-diméthylpropylammonium; hexanoate de N,N-diméthylpropylammonium; heptanoate de N,N-diméthylpropylammonium; octanoate de N,N-diméthylpropylammonium; formiate de N,N-diméthylbutylammonium; valérate de N,N-diméthylhexylammonium; butanoate de N,N-diméthyldécylammonium; valérate de N,N-diméthyldécylammonium; hexanoate de N,N-diméthyldécylammonium; heptanoate de N,N-diméthyldécylammonium; octanoate de N,N-diméthyldécylammonium; pivalate de N,N-diméthyldécylammonium; valérate de N,N-diméthyldodécylammonium; acétate de N-méthyldiéthylammonium; propanoate de N-méthyldiéthylammonium; butanoate de N-méthyldiéthylammonium; valérate de N-méthyldiéthylammonium; hexanoate de N-méthyldiéthylammonium; heptanoate de N-méthyldiéthylammonium; octanoate de N-méthyldiéthylammonium; acétate de N-méthyldipropylammonium; propanoate de N-méthyldipropylammonium; butanoate de N-méthyldipropylammonium; valérate de N-méthyldipropylammonium; hexanoate de N-méthyldipropylammonium; heptanoate de N-méthyldipropylammonium; octanoate de N-méthyldipropylammonium; acétate de N-méthyldibutylammonium; propanoate de N-méthyldibutylammonium; butanoate de N-méthyldibutylammonium; valérate de N-méthyldibutylammonium; hexanoate de N-méthyldibutylammonium; heptanoate de N-méthyldibutylammonium; octanoate de N-méthyldibutylammonium; propanoate de N-méthyldicyclohexylammonium; butanoate de N-méthyldicyclohexylammonium; valérate de N-méthyldicyclohexylammonium; hexanoate de N-méthyldicyclohexylammonium; heptanoate de N-méthyldicyclohexylammonium; octanoate de N-méthyldicyclohexylammonium; formiate de N-éthyldiisopropylammonium; valérate de N-éthyldiisopropylammonium; hexanoate de N-éthyldiisopropylammonium; heptanoate de N-éthyldiisopropylammonium; 2-méthylvalérate de N-éthyldiisopropylammonium; 2-hexyldécanoate de N-éthyldiisopropylammonium; valérate de triéthylammonium; heptanoate de triéthylammonium; octanoate de triéthylammonium; méthanesulfonate de triéthylammonium; propanoate de tripropylammonium; butanoate de tripropylammonium; valérate de tripropylammonium; hexanoate de tripropylammonium; heptanoate de tripropylammonium; octanoate de tripropylammonium; propanoate de tributylammonium; butanoate de tributylammonium; valérate de tributylammonium; hexanoate de tributylammonium; heptanoate de tributylammonium; octanoate de tributylammonium; valérate de triisobutylammonium; hexanoate de triisobutylammonium; heptanoate de triisobutylammonium; octanoate de triisobutylammonium; crotonate de triisobutylammonium; 2,2-diméthylbutanoate de triisobutylammonium; 2-méthylvalérate de triisobutylammonium; décanoate de triisobutylammonium; butanoate de trihexylammonium; valérate de trihexylammonium; hexanoate de trihexylammonium; heptanoate de trihexylammonium; octanoate de trihexylammonium; acétate de trioctylammonium; butanoate de trioctylammonium; valérate de trioctylammonium; hexanoate de trioctylammonium; heptanoate de trioctylammonium; octanoate de trioctylammonium; nonanoate de trioctylammonium; décanoate de trioctylammonium; crotonate de trioctylammonium; trans-2-hexénoate de trioctylammonium; 2-butyloctanoate de trioctylammonium; benzoate de trioctylammonium; phenylacétate de trioctylammonium; propanoate de triisooctylammonium; butanoate de triisooctylammonium; valérate de triisooctylammonium; hexanoate de triisooctylammonium; heptanoate de triisooctylammonium; octanoate de triisooctylammonium; formiate de N-méthylpipéridinium; valérate de N-méthylpipéridinium; formiate de N-méthylpyrrolidinium; acétate de N-méthylpyrrolidinium; propanoate de N-méthylpyrrolidinium; butanoate de N-méthylpyrrolidinium; valérate de N-méthylpyrrolidinium; hexanoate de N-méthylpyrrolidinium; heptanoate de N-méthylpyrrolidinium; et octanoate de N-méthylpyrrolidinium.

2. Utilisation selon la revendication 1 dans laquelle le groupement trialkylammonium est du triéthylammonium.

3. Utilisation selon la revendication 2 dans laquelle le sel de trialkylammonium est du propanoate de triéthylammonium.

4. Utilisation selon la revendication 1 dans laquelle le solvant est un solvant d'extraction.

5. Utilisation selon la revendication 4 dans laquelle le solvant est utilisé dans l'extraction d'un produit naturel.

6. Utilisation selon la revendication 5 dans laquelle le produit naturel est un terpène.

7. Méthode de fabrication d'un composé organique qui comprend l'utilisation d'un liquide ionique protique contenant un sel de trialkylammonium comme solvant dans laquelle le groupement trialkylammonium est sélectionné à partir de groupes composés d'acétate de diméthyléthylammonium; propanoate de N,N-diméthyléthylammonium; butanoate de N,N-diméthyléthylammonium; hexanoate de N,N-diméthyléthylammonium; heptanoate de N,N-diméthyléthylammonium; octanoate de N,N-diméthyléthylammonium; crotonate de N,N-diméthyléthylammonium; phenylacétate de N,N-diméthyléthylammonium; 2,2-diméthylbutanoate de N,N-diméthyléthylammonium; acétate de N,N-diméthylbutylammonium; propanoate de N,N-diméthylbutylammonium; butanoate de N,N-diméthylbutylammonium; hexanoate de N,N-diméthylbutylammonium; heptanoate de N,N-diméthylbutylammonium; octanoate de N,N-diméthylbutylammonium; benzoate de N,N-diméthylbutylammonium; phenylacétate de N,N-diméthylbutylammonium; 2,2-diméthylbutanoate de N,N-diméthylbutylammonium; 2-hexyldécanoate de N,N-diméthylbutylammonium; 2-méthylvalérate de N,N-diméthylbutylammonium; décanoate de N,N-diméthylbutylammonium; acétate de N,N-diméthylhexylammonium; propanoate de diméthyldodécylammonium; butanoate de N,N-diméthyldodécylammonium; hexanoate de N,N-diméthyldodécylammonium; heptanoate de N,N-diméthyldodécylammonium; octanoate de N,N-diméthyldodécylammonium; acétate de N-méthylpipéridinium; propanoate de N-méthylpipéridinium; butanoate de N-méthylpipéridinium; hexanoate de N-méthylpipéridinium; heptanoate de N-méthylpipéridinium; propanoate de N,N,N-triéthylammonium; butanoate de N,N,N-triéthylammonium; hexanoate de N,N,N-triéthylammonium; crotonate de N,N,N-triéthylammonium; butanoate de N,N-diisopropyl-N-éthylammonium; octanoate de N,N-düsopropyl-N-éthylammonium; propanoate de N,N,N-trioctylammonium; 2-oxobutanoate de N,N,N-trioctylammonium; 3-toluate de N,N,N-trioctylammonium; butanoate de N,N,N-triisobutylammonium; formiate de triméthylammonium; acétate de triméthylammonium; propanoate de triméthylammonium; butanoate de triméthylammonium; valérate de triméthylammonium; hexanoate de triméthylammonium; heptanoate de triméthylammonium; octanoate de triméthylammonium; glycolate de triméthylammonium; formiate de N,N-diméthyléthylammonium; valérate de N,N-diméthyléthylammonium; trans-2-hexénoate de N,N-diméthyléthylammonium; formiate de N,N-diméthylpropylammonium; acétate de N,N-diméthylpropylammonium; propanoate de N,N-diméthylpropylammonium; butanoate de N,N-diméthylpropylammonium; valérate de N,N-diméthylpropylammonium; hexanoate de N,N-diméthylpropylammonium; heptanoate de N,N-diméthylpropylammonium; octanoate de N,N-diméthylpropylammonium; formiate de N,N-diméthylbutylammonium; valérate de N,N-diméthylhexylammonium; butanoate de N,N-diméthyldécylammonium; valérate de N,N-diméthyldécylammonium; hexanoate de N,N-diméthyldécylammonium; heptanoate de N,N-diméthyldécylammonium; octanoate de N,N-diméthyldécylammonium; pivalate de N,N-diméthyldécylammonium; valérate de N,N-diméthyldodécylammonium; acétate de N-méthyldiéthylammonium; propanoate de N-méthyldiéthylammonium; butanoate de N-méthyldiéthylammonium; valérate de N-méthyldiéthylammonium; hexanoate de N-méthyldiéthylammonium; heptanoate de N-méthyldiéthylammonium; octanoate de N-méthyldiéthylammonium; acétate de N-méthyldipropylammonium; propanoate de N-méthyldipropylammonium; butanoate de N-méthyldipropylammonium; valérate de N-méthyldipropylammonium; hexanoate de N-méthyldipropylammonium; heptanoate de N-méthyldipropylammonium; octanoate de N-méthyldipropylammonium; acétate de N-méthyldibutylammonium; propanoate de N-méthyldibutylammonium; butanoate de N-méthyldibutylammonium; valérate de N-méthyldibutylammonium; hexanoate de N-méthyldibutylammonium; heptanoate de N-méthyldibutylammonium; octanoate de N-méthyldibutylammonium; propanoate de N-méthyldicyclohexylammonium; butanoate de N-méthyldicyclohexylammonium; valérate de N-méthyldicyclohexylammonium; hexanoate de N-méthyldicyclohexylammonium; heptanoate de N-méthyldicyclohexylammonium; octanoate de N-méthyldicyclohexylammonium; formiate de N-éthyldiisopropylammonium; valérate de N-éthyldiisopropylammonium; hexanoate de N-éthyldiisopropylammonium; heptanoate de N-éthyldiisopropylammonium; 2-méthylvalérate de N-éthyldiisopropylammonium; 2-hexyldécanoate N-éthyldiisopropylammonium; valérate de triéthylammonium; heptanoate de triéthylammonium; octanoate de triéthylammonium; méthanesulfonate de triéthylammonium; propanoate de tripropylammonium; butanoate de tripropylammonium; valérate de tripropylammonium; hexanoate de tripropylammonium; heptanoate de tripropylammonium; octanoate de tripropylammonium; propanoate de tributylammonium; butanoate de tributylammonium; valérate de tributylammonium; hexanoate de tributylammonium; heptanoate de tributylammonium; octanoate de tributylammonium; valérate de triisobutylammonium; hexanoate de triisobutylammonium; heptanoate de triisobutylammonium; octanoate de triisobutylammonium; crotonate de triisobutylammonium; 2,2-diméthylbutanoate de triisobutylammonium; 2-méthylvalérate de triisobutylammonium; décanoate de triisobutylammonium; butanoate de trihexylammonium; valérate de trihexylammonium; hexanoate de trihexylammonium; heptanoate de trihexylammonium; octanoate de trihexylammonium; acétate de trioctylammonium; butanoate de trioctylammonium; valérate de trioctylammonium; hexanoate de trioctylammonium; heptanoate de trioctylammonium; octanoate de trioctylammonium; nonanoate de trioctylammonium; décanoate de trioctylammonium; crotonate de trioctylammonium; trans-2-hexénoate de trioctylammonium; 2-butyloctanoate de trioctylammonium; benzoate de trioctylammonium; phenylacétate de trioctylammonium; propanoate de triisooctylammonium; butanoate de triisooctylammonium; valérate de triisooctylammonium; hexanoate de triisooctylammonium; heptanoate de triisooctylammonium; octanoate de triisooctylammonium; formiate de N-méthylpipéridinium; valérate de N-méthylpipéridinium; formiate de N-méthylpyrrolidinium; acétate de N-méthylpyrrolidinium; propanoate de N-méthylpyrrolidinium; butanoate de N-méthylpyrrolidinium; valérate de N-méthylpyrrolidinium; hexanoate de N-méthylpyrrolidinium; heptanoate de N-méthylpyrrolidinium; et octanoate de N-méthylpyrrolidinium.

8. Méthode selon la revendication 7 dans laquelle le groupement trialkylammonium est du triéthylammonium.

9. Méthode selon la revendication 7 dans laquelle l'anion du sel est un acide organique et l'acide organique est un acide carboxylique.

10. Méthode selon la revendication 9 dans laquelle l'acide organique est un acide carboxylique avec un nombre de carbones de 1 à 10.

11. Méthode selon la revendication 10 dans laquelle le sel de l'acide organique est sélectionné à partir de groupes composés de formiate, acétate, propanoate et butanoate.

12. Méthode selon la revendication 8 dans laquelle le sel de trialkylammonium est du propanoate de triéthylammonium.

13. Méthode selon la revendication 7 dans laquelle la méthode comprend l'extraction d'un produit naturel.

14. Méthode selon la revendication 13 dans laquelle le produit naturel est un terpène.
